# EUROPÄISCHE PATENTSCHRIFT

(11) **EP 0 930 869 B1**
(45) Veröffentlichungstag und Bekanntmachung des Hinweises auf die Patenterteilung: **15.05.2002**
(21) Anmeldenummer: 97912138.1
(22) Anmeldetag: 08.10.1997
(51) Int. Cl.: A61K 7/38, A61K 7/32

(54) **DESODORIERENDE ZUBEREITUNGEN**
DEODORIZING PREPARATIONS
PREPARATIONS DESODORISANTES

(30) Priorität: 17.10.1996 DE 19642874
(43) Veröffentlichungstag der Anmeldung: 28.07.1999
(73) Patentinhaber: Cognis Deutschland GmbH & Co. KG, 40589 Düsseldorf (DE)
(72) Erfinder: WACHTER, Rolf, D-40595 Düsseldorf (DE); TESMANN, Holger, D-41363 Jüchen (DE); BEHLER, Ansgar, D-46240 Bottrop (DE); MAURER, Karl-Heinz, D-40699 Erkrath (DE)
(86) Internationale Anmeldenummer: EP9705523
(87) Internationale Veröffentlichungsnummer: WO9817241

(56) Entgegenhaltungen:
- DE-A- 4 107 712
- FR-A- 2 597 367
- GB-A- 2 029 441

## Beschreibung

### Gebiet der Erfindung

Die Erfindung betrifft desodorierende Zubereitungen mit einem Gehalt an Sterolsulfaten, Aluminiumchlorhydrat, Esteraseinhibitoren und bakteriziden/bakteriostatischen Wirkstoffen sowie die Verwendung von Sterolsulfaten zur Herstellung von desodorierenden Zubereitungen

### Stand der Technik

Im Bereich der Körperpflege werden Desodorantien zur Beseitigung störender Körpergerüche eingesetzt. Diese entstehen bei der bakteriellen Zersetzung des an sich geruchlosen Schweißes, insbesondere in den feuchtwarmen Achselhöhlen oder unter ähnlichen, den Mikroorganismen gute Lebensbedingungen bietenden Bedingungen. Körpergerüche können durch geeignete Riechstoffe überdeckt werden. Man kann sie auch bekämpfen, indem man Präparate einsetzt, die die Schweißabsonderung selbst hemmen oder die Zersetzung des Schweißes inhibieren (sogenannte Antihidrotika, Antiperspirantien oder Antitranspirantien). Typische Beispiele für derartige Substanzen sind Aluminiumverbindungen wie Aluminiumsulfat oder Aluminiumchlorhydrat, Zinksalze und Citronensäureverbindungen. Eine Übersicht hierzu findet sich beispielsweise in **Umbach (Hrsg.), "Kosmetik", S.141f., Thieme Verlag, Stuttgart, 1988.**

GB-A-20 29441 offenbast desodorierende zusammensetzungen die Cortison-Sulfat enthalten.

Aus der täglichen Lebenserfahrung ist jedoch klar, daß das Problem der Geruchsinhibierung, insbesondere bei Hitze oder körperlicher Betätigung keineswegs vollständig gelöst ist. Die Produkte des Marktes vermögen weder die Absonderung von Schweiß noch die Bildung von Gerüchen dauerhaft zu unterbinden. Vielmehr ist die Inhibierung zeitlich begrenzt und auch davon abhängig, in welchem Umfang Schweiß abgesondert wird. Demzufolge besteht ein andauerndes Bedürfnis nach Produkten, die hinsichtlich der Minimierung der Schweißabsonderung und der Verminderung der Geruchsbildung verbessert sind und dabei gleichzeitig noch eine erhöhte hautkosmetische Verträglichkeit, d.h. ein vermindertes Irritationspotential gegenüber besonders empfindlicher Haut aufweisen. Die Aufgabe der Erfindung hat somit darin bestanden, derartige Produkte zur Verfügung zu stellen.

### Beschreibung der Erfindung

Gegenstand der Erfindung sind desodorierende Zubereitungen, enthaltend
(a) Sterolsulfate,
(b1) Aluminiumchlorhydrat,
(b2) Esteraseinhibitoren und/oder
(b3) bakterizide bzw. bakteriostatische Wirkstoffe.

Die Verwendung von Aluminiumchlorhydraten, Esteraseinhibitoren (z.B. Triethylcitrat) und bakteriziden Wirkstoffen (z.B. Chitosan) zur Herstellung von desodorierenden und/oder schweißhemmenden Zusammensetzungen ist aus dem Stand der Technik bekannt. Überraschenderweise wurde gefunden, daß Sterolsulfate die Aktivität esterolytischer Enzyme bereits im unteren ppm-Bereich inhibieren und zusammen mit den vorgenannten Komponenten eine synergistische desodorierende Wirkung erzielt wird. Die Sterolsulfate wirken selektiv auf Serinesterasen bzw. Serinproteasen ohne das biologische Gleichgewicht der Hautflora zu beeinträchtigen. Gleichzeitig führt der Einsatz der Sterolsulfate zu einer Verbesserung der hautkosmetischen Verträglichkeit der Produkte.

### Sterolsulfate

Sterolsulfate stellen bekannte Stoffe dar, die beispielsweise durch Sulfatierung von Sterinen mit einem Komplex aus Schwefeltrioxid und Pyridin in Benzol hergestellt werden können [vgl. **J.Am.Chem.Soc. 63, 1259 (1941)]**. Unter Sterinen, die als Einsatzstoffe zur Herstellung der Sterolsulfate in Betracht kommen, sind solche Steroide zu verstehen, die nur am C-3 eine Hydroxylgruppe, sonst aber keine funktionellen Gruppen tragen. Es handelt sich also formal um Alkohole, weswegen diese Gruppe von Verbindungen auch gelegentlich als Sterole bezeichnet werden. In der Regel besitzen die Sterine 27 bis 30 Kohlenstoffatome und eine Doppelbindung in 5/6, gegebenenfalls 7/8, 8/9 oder anderen Positionen. Als Ausgangsstoffe kommen jedoch neben diesen ungesättigten Spezies auch die durch Härtung erhältlichen gesättigten Verbindungen in Frage. Typische Beispiele für geeignete Sterolsulfate sind solche auf Basis von Zoosterinen wie etwa tierisches Cholesterin, Lanosterinen aus Wollfett, Spongosterinen aus Schwämmen oder Stellasterinen aus Seestemen. Wegen der helleren Farbe der Sulfatierungsprodukte werden jedoch vorzugsweise Phytosterolsulfate eingesetzt, wie beispielsweise solche auf Basis von Ergosterinen, Campesterinen, Stigmasterinen und Sistosterinen.

### Aluminiumchlorhydrat

Bei Aluminiumchlorhydaten der Komponente (b1) handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über ein Zusammenziehen bzw. Verkleben der Schweißdrüsen durch Eiweißfällung und/oder Feuchtigkeitsentzug [vgl. **J.Soc.Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J. Pharm.Pharmacol. 26, 531 (1975)].**

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Esteraseinhibitoren der Komponente (b2), vorzugsweise Trialkylcitrate wie Trimethylcitrat, Tripropylcitrat, Tributylcitrat und insbesondere Triethylcitrat (Hydagen® CAT, Henkel KGaA, Düssel-dorf/FRG) inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme durch Acylierung inaktiviert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester.

### Bakterizide bzw. bakteriostatische Wirkstoffe

Typische Beispiele für geeignete bakterizide bzw. bakteriostatische Wirkstoffe der Komponente (b3) sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

### Gewerbliche Anwendbarkeit

Sterolsulfate haben sich für den beschriebenen Anwendungszweck enzyminhibierend erwiesen. Ein weiterer Gegenstand der Erfindung betrifft daher ihre Verwendung, alleine oder in Abmischung mit Aluminiumchlorhydraten, weiteren Esteraseinhibitoren und/oder bakteriziden bzw. bakteriostatischen Wirkstoffen, zur Herstellung von desodorierenden Zubereitungen.

Die Zusammensetzungen können in einer bevorzugten Ausführungsform der Erfindung die Komponenten (a) und (b) vorzugsweise in den folgenden Mengen - bezogen auf den Feststoffanteil - enthalten:
(a) 0,01 bis 50, vorzugsweise 0,1 bis 5 Gew.-% Sterolsutfate,
(b1) 1 bis 50, vorzugsweise 10 bis 50 Gew.-% Aluminiumchlorhydrat,
(b2) 0,01 bis 20, vorzugsweise 1 bis 5 Gew.-% Esteraseinhibitoren und
(b3) (d) 0,01 bis 5, vorzugsweise 0,1 bis 1 Gew.-% bakterizide bzw. bakteriostatische Wirkstoffe.

Hierbei besteht die Maßgabe, daß sich die Mengenangaben zu 100 Gew.-% ergänzen. Die Angaben verstehen sich jeweils auf den Aktivsubstanzgehalt der Komponenten.

### Keimhemmende Mittel

Die erfindungsgemäßen Zubereitungen können als weitere Zusatzstoffe bekannte keimhemmende Mittel enthalten. Typische Beispiele sind Konservierungsmittel mit spezifischer Wirkung gegen grampositive Bakterien wie etwa 2,4,4'-Trichlor-2'-hydroxydiphenylether, Chlorhexidin (1,6-Di-(4-chlorphenylbiguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

### Hilfs- und Zusatzstoffe

Um die Wirkstoffe auf eine dosierbare, sparsame, bequeme und kosmetisch ansprechende Weise auf die Haut applizieren zu können, werden sie üblicherweise in Rezepturgrundlagen eingearbeitet. Als wichtigste Grundlagen sind zu nennen: Alkoholische und wäßrig/alkoholische Lösungen, Emulsionen, Gele, Öle, Wachs/Fett-Massen, Stiftpräparate und Puder. So können die erfindungsgemäßen Zubereitungen beispielsweise jeweils bis zu 60 Gew.-% niedere aliphatische Alkohole, vorzugsweise Ethanol sowie organische Säuren wie z.B. Glycolsäure enthalten. Weitere Einsatzstoffe sind Überfettungsmittel, Emulgatoren, Antioxidantien, Talkum, Kieselsäure (z.B. als Träger für das Aluminiumchlorhydrat), sowie Parfumöle, etherische Öle, Farbstoffe und - für Sprayanwendungen - Treibgase wie beispielsweise Propan und/oder Butan. Die Mittel kommen vorzugsweise als Roller (Roll-on-Emulsionen), Stifte, Deo-oder Pumpsprays in den Handel.

### Beispiele

Die Wirksamkeit der erfindungsgemäßen Mittel wurde stellvertretend über die Esteraseinhibierung bestimmt. Hierzu wurde nach 15 minütiger Einwirkzeit von 2000 ppm der Testgemische auf die Esterase bei pH = 6 deren Restaktivität parallel zu einer ungehemmten Esterase bestimmt (Standard = 100 %). Die Zusammensetzungen 1 bis 6 sind erfindungsgemäß, die Rezepturen V1 bis V3 dienen zum Vergleich. Die Ergebnisse sind in Tabelle 1 zusammengefaßt (Mengenangaben als Gew.-%).

**Tabelle 1**

| **Rezepturen und Esteraseinhibierung** | | | | | | | | | |
|---|---|---|---|---|---|---|---|---|---|
| **Komponenten** | **1** | **2** | **3** | **4** | **5** | **6** | **V1** | **V2** | **V3** |
| Sterolsulfat-Natriumsalz | 4 | 4 | 4 | 4 | 4 | 4 | - | - | - |
| Aluminiumchlorhydrat | - | 50 | - | 50 | 10 | 50 | 50 | - | 50 |
| Triethylcitrat | - | - | 5 | 6 | 5 | 3 | - | 5 | 5 |
| Chitosan | - | - | - | - | - | 3 | - | - | - |
| Ethanol | 20 | - | 20 | 20 | 20 | 10 | 20 | 20 | 20 |
| Glycolsäure | 0,016 | 0,016 | 0,016 | 0,016 | 0,016 | 0,016 | - | - | - |
| Famesol | - | - | - | | 1 | - | - | - | - |
| Wasser | ad 100 | | | | | | | | |
| ***Esterase-Aktivität [%]*** | 78 | 73 | 75 | 30 | 28 | 27 | 100 | 77 | 75 |

## Patentansprüche

1. Desodorierende Zubereitungen, enthaltend
(a) Sterolsulfate,
(b1) Aluminiumchlorhydrat,
(b2) Esteraseinhibitoren und/oder
(b3) bakterizide bzw. bakteriostatische Wirkstoffe,
**dadurch gekennzeichnet, dass** das Steroidgerüst der Sterolsulfate (a) mit Ausnahme der Position am C 3 keine weiteren funktionellen Gruppen trägt.

2. Zubereitungen nach Anspruch 1, **dadurch gekennzeichnet, dass** sie Phytosterolsulfate enthalten

3. Zubereitungen nach den Ansprüchen 1 und 2, **dadurch gekennzeichnet, dass** sie als Esteraseinhibitoren Trialkylcitrate enthalten.

4. Zubereitungen nach den Ansprüchen 1 bis 3, **dadurch gekennzeichnet, dass** sie als bakterizide bzw. bakteriostatische Wirkstoffe Chitosane enthalten.

5. Zubereitungen nach den Ansprüchen 1 bis 4, **dadurch gekennzeichnet, dass** sie - bezogen auf den Feststoffgehalt -
(a) 0,01 bis 5 Gew.-% Sterolsulfate,
(b) 1 bis 50 Gew.-% Aluminiumchlorhydrat und
(c) 0,01 bis 20 Gew.-% Esteraseinhibitoren
enthalten, mit der Maßgabe, dass sich die Mengenangaben zu 100 Gew.-% ergänzen.

6. Verwendung von Sterolsulfaten, deren Steroidgerüst mit Ausnahme der Position am C 3 keine weiteren funktionellen Gruppen trägt, zur Herstellung von desodorierenden Zubereitungen.

## Claims

1. Deodorizing preparations containing
(a) sterol sulfates,
(b1) aluminium chlorohydrate,
(b2) esterase inhibitors and/or
(b3) bactericidal or bacteriostatic agents,
**characterized in that** the steroid skeleton of the sterol sulfates (a) contains no other functional groups except at C 3.

2. Preparations as claimed in claim 1, **characterized in that** they contain phytosterol sulfates.

3. Preparations as claimed in claims 1 and 2, **characterized in that** they contain trialkyl citrates as esterase inhibitors.

4. Preparations as claimed in claims 1 to 3, **characterized in that** they contain chitosans as bactericidal or bacteriostatic agents.

5. Preparations as claimed in claims 1 to 4, **characterized in that**, based on their solids content, they contain
(a) 0.01 to 5% by weight of sterol sulfates,
(b) 1 to 50% by weight of aluminium chlorohydrate and
(c) 0.01 to 20% by weight of esterase inhibitors,
with the proviso that the quantities shown add up to 100% by weight.

6. The use of sterol sulfates of which the steroid skeleton contains no other functional groups except at C 3 for the production of deodorizing preparations.

## Revendications

1. Préparations désodorisantes contenant :
a) des sulfates de stérol
b1) du chlorhydrate d'aluminium
b2) des inhibiteurs d'estérase et/ou
b3) des principes actifs bactéricides ou bactériostatiques
**caractérisées en ce que**
le noyau stéroïde des sulfates de stérol a) ne porte aucun autre groupe fonctionnel à l'exception de la position en C₃.

2. Préparations selon la revendication 1,
**caractérisées en ce qu'**
elles contiennent des sulfates de phytostérol.

3. Préparations selon les revendications 1 et 2,
**caractérisées en ce qu'**
elles contiennent en tant qu'inhibiteur d'estérase, un citrate de trialkyle.

4. Préparations selon les revendications 1 à 3,
**caractérisées en ce qu'**
elles contiennent du chitosane comme principes actifs bactéricides ou bactériostatiques.

5. Préparations selon les revendications 1 à 4,
**caractérisées en ce qu'**
elles contiennent - rapporté à la teneur en matières solides
a) de 0,01 à 5 % en poids de sulfates de stérol
b) de 1 à 50 % en poids de chlorhydrate d'aluminium
c) de 0,01 à 20 % en poids d'inhibiteurs d'estérase avec la précision que les données en quantités se complètent à 100 % en poids.

6. Utilisation des sulfates de stérol dont le noyau stérol ne porte aucun autre groupe fonctionnel à l'exception de la position en C₃, en vue de la production de préparations désodorisantes.
